**Europäisches Patentamt**

**European Patent Office**

**Office européen des brevets**

(11) Veröffentlichungsnummer: **0 296 421 B1**

## EUROPÄISCHE PATENTSCHRIFT

(45) Veröffentlichungstag der Patentschrift: **22.04.92**

(21) Anmeldenummer: **88109256.3**

(22) Anmeldetag: **10.06.88**

(51) Int. Cl.⁵: **A61B 10/00**

(54) Biopsienadel.

(30) Priorität: **26.06.87 DE 8708860 U**

(43) Veröffentlichungstag der Anmeldung:
**28.12.88 Patentblatt 88/52**

(45) Bekanntmachung des Hinweises auf die
Patenterteilung:
**22.04.92 Patentblatt 92/17**

(84) Benannte Vertragsstaaten:
**AT BE CH DE ES FR GB IT LI LU NL**

(56) Entgegenhaltungen:
**WO-A-83/02220**
**FR-A- 2 272 633**
**US-A- 3 850 158**
**US-A- 4 403 617**

(73) Patentinhaber: **ANGIOMED Aktiengesellschaft**
**Eisenbahnstrasse 36**
**W-7500 Karlsruhe 41(DE)**

(72) Erfinder: **Schnepp-Pesch, Wolfram**
**Schönblick 6**
**W-7505 Ettlingen(DE)**
Erfinder: **Lindenberg, Josef**
**Käthe-Kollwitz-Strasse 10a**
**W-7500 Karlsruhe(DE)**

(74) Vertreter: **Dr.-Ing. Hans Lichti Dipl.-Ing. Heiner**
**Lichti Dipl.-Phys. Dr. Jost Lempert**
**Postfach 41 07 60 Bergwaldstrasse 1**
**W-7500 Karlsruhe 41(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Biopsienadel zur Knochenbiopsie, insbesondere durch Einbringen der Nadel in und gegebenenfalls durch die Knochengrundsubstanz, mit einer distal stirnseitig geschliffenen Kanüle und einem in dieser geführten Stilett, wobei die Kanüle an ihrem distalen Ende ein Außengewinde aufweist.

Instrumente zur Knochenbiopsie sind bekannt. Sie weisen ein Hohlrohr und einen in dieses geführten Obturator, der distal etwas aus dem Hohlrohr hinausragt, auf. Das Hohlrohr wird mit eingeführtem Obturator in die Knochengrundsubstanz, insbesondere auch die Substantia corticalis sive compacta eingetrieben. Hierzu wird zum Teil ein Biopsiehammer verwendet, mit dem die Biopsienadel in den Knochen eingetrieben wird. Nach Entfernen des Obturators muß das Hohlrohr weiter eingetrieben werden, um das Knochenmaterial aufzunehmen. Aufgrund der Härte des Knochens werden zum Teil bewußt, zum Teil unbewußt seitliche Bewegungen des Hohlrohrs ausgeführt, da dieses nicht geführt ist, auch wenn, wie vorgeschlagen wurde, ein konischer Endabschnitt als Raspel aufgerauht oder mit einem Gewinde versehen ist, um das Eindringen in den Knochen - und durch Drehen - zu erleichtern. Hierdurch werden relativ große Bereiche um den eigentlichen Stichkanal herum beschädigt. Aufgrund der mangelnden Führung der hierdurch bedingten Kippkräfte ist erforderlich, daß das Hohlrohr eine hohe Eigenstabilität aufweist. Es hat daher nicht nur einen großen Außendurchmesser von ca. 8 mm, sondern auch relativ starke Mantelwandstärken im Bereich von 2 mm und u.a. deswegen, aber auch weil man bisher der Meinung war, daß ein relativ großer Materialpropfen zu entnehmen ist, relativ große Innendurchmesser von 4 mm, so daß die Ausstanzung eines gewünschten Knochenzylinders von etwa 4 mm Durchmesser mit einem solchen Instrument zur histologischen Untersuchung von Mark und umgebenden Knochen gesichert war (US-PS 3 850 158). Bei einem Hohlrohr mit einem scharfen, mit Graten versehenen Gewinde könnte sich beim Eindrehen einerseits Muskelfleisch und Gewebe auf dem Gewinde aufwickeln, andererseits, wei festgestellt wurde, beim Herausziehen der Knochen splittern. Beides kann zu erheblichen Traumen führen, ebenso wie die Stärke des Instruments selbst.

Weiterhin sind Punktionsnadeln mit geringem Durchmesser zur Punktion weicher Gewebe bekannt.

Der Erfindung liegt die Aufgabe zugrunde eine gattungsgemäße Biopsienadel zur Knochenbiopsie derart auszugestalten, daß unter Vermeidung der vorgenannten Nachteile eine einfachere, weniger Körpermaterial beschädigende und dem Patienten weniger belastende Knochenbiopsie möglich ist.

Erfindungsgemäß wird die genannte Aufgabe durch eine Biopsienadel der eingangs genannten Art gelöst, bei der das Außengewinde derart überschliffen ist, daß die Wandungen zweier benachbarter Gewindeteile oder -nuten nicht über eine Gewinde-Schneidkante ineinander übergehen, sondern über einen Außenmantelbereich mit endlicher Erstrekkung in axialer Richtung, dessen Außendurchmesser nicht größer als der Außendurchmesser der sonstigen, im wesentlichen zylindrischen Kanülen ist.

Durch die Ausbildung eines überschliffenen Gewindes auf dem Außenumfang der Kanüle kann die Kanüle nicht nur mittels eines fest mit ihr verbundenen oder an ihr anzubringenden Drehgriff unter Drehung selbstschneidend in das Knochenmaterial eingedreht werden, sondern durch die überschliffene Ausgestaltung des Gewindes wird erreicht, daß beim Eindrehen Muskelfleisch und Gewebe aufgrund der fehlenden Grate über das Winde freigleiten und sich nicht auf diesem aufwikkeln. Das überschliffene Gewinde weist, wie gesagt, keine Grate auf. Erfindungsgemäß gehen die Wandungen zweier benachbarter Gewindeteile oder -nuten nicht über eine Gewinde-Schneidkante ineinander über, sondern über einen Außenmantelbereich mit endlicher Erstreckung in axialer Richtung, dessen Außendurchmesser nicht größer als der Außendurchmesser der sonstigen, im wesentlichen zylindrischen Kanülen ist, im Gegensatz zu einem nicht überschliffenen Gewinde nach dem Stand der Technik, bei dem die Gewindegrate durch die Gewindeherstellung über den Umfang des entsprechenden Bereichs vor Gewindeerzeugung hinausgedrückt werden. Durch die überschliffene Ausbildung wird weiterhin ein einfaches Herausziehen der Kanüle nach Aufnahme eines Materialpropfens erreicht, ohne daß ein Splittern des Knochens zu befürchten ist. Durch eine zylindrische Ausgestaltung des Gewindes wird neben Unterstützung der vorgenannten Vorteile insbesondere erreicht, daß von außen weniger axiale Druckkräfte aufzubringen sind, die leicht zu Kippbewegungen und daher zu Beschädigungen und Belastungen führen können. Darüberhinaus wird die Kanüle durch das zylindrische Gewinde sicher weitgehend axial geführt werden, was durch Gewinde mit einer Windungszahl im Bereich von weniger als zehn, insbesondere schon bei drei bis zu sechs Windungen gut erreichbar ist. Das Gewinde kann insbesondere auch als Einfach oder Mehrfachwendel, wie als Doppelwendel ausgeführt sein. Durch den hierdurch gesicherten axialen Vorschub der Kanüle kann eine zylindrische Materialprobe ausgeschnitten werden, ohne daß diese durch Verkantungen seitlichen Quetschungen und dergleichen ausgesetzt ist. Dies wird insbesondere durch einen vorteilhaften, motorischen Antrieb unterstützt, bei dem die Gefahr eines Verkantens, wie es bei einem Eindrehen von Hand aufgrund der Notwendigkeit des wiederholten "Nachfassens" am Handgriff gegeben ist, ebenso weiter reduziert wird, wie durch den Wegfall der Notwendigkeit des Drehens von Hand an sich, so daß sich der Operateur voll auf die axiale Führung konzentrieren kann, da der Drehantrieb durch den Motor erfolgt.

Eine sehr bevorzugte Ausgestaltung sieht vor, daß das Stilett eine distale Spitze mit Trokarschliff aufweist. Hierdurch wird eine Führung der Biopsienadel beim Einstellen durch das weiche Gewebe unterstützt, während beim Stand der Technik aufgrund der keilartigen Ausbildung des Obturators in Grenzbereichen unterschiedlich festen Gewebes das Instrument leicht zur Seite gedrückt werden kann bzw. um dies zu erreichen, seitliche (Kipp-)kräfte aufgebracht werden müssen, die wiederum Traumata bedingen.

Die Materialstärke der Kanüle kann wesentlich geringer sein, als dies bisher der Fall war und der Innendurchmesser und damit auch der Gesamtdurchmesser der Kanüle können geringer gewählt werden, was die Möglichkeit des motorischen Antriebs unterstützt. Zur Möglichkeit einer geringeren Wahl des Gesamtdurchmessers der Kanüle trägt auch bei, daß aufgrund der Vermeidung der Kipp- oder Knickbewegungen durch die axiale Gewindeführung, aber auch durch eine relativ kurze Ausführung der Kanüle mit einem freien Erstreckungsbereich deutlich unter 100 mm (bei einer Gesamtlänge bis zu 140 mm, vorzugsweise unter 60 mm, die zum Teil ein proximales Adapter- oder Ansatzstück einschließt), die Biege- oder Knickstabilität der Kanüle selbst ohne Gefahr reduziert werden kann. Hierdurch kann einerseits der Gesamtaußendurchmesser der Kanüle als solcher, darüberhinaus aber auch die Wandstärke der Kanüle reduziert werden. In bevorzugter Ausgestaltung ist daher weiterhin vorgesehen, daß der Außendurchmesser der Kanüle kleiner als 2 mm ist, wobei insbesondere der Außendurchmesser zwischen 1 und 1,5 mm, der Innendurchmesser zwischen 0,7 und 1,2 sowie die Wandstärke der Kanüle zwischen 0,1 und 0,2 mm liegt.

Die Entnahme des Materialpfropfens wird weiterhin dadurch unter stützt, daß in der Kanüle ein Unterdruck ausgebildet wird, welcher den Pfropfen beim Herausziehen der Kanüle in dieser hält. Hierdurch wird die Notwendigkeit des Abbrechens eines aus einem Knochen um seinen Umfang herumgeschnittenen Pfropfens, der aber an seiner Stirnseite noch am Restknochen hängt, vermieden, was beim Stand der Technik ebenfalls nur durch ein Verkippen mit der hierdurch bedingten Traumenbildung erreichbar ist. Die Ablösung des herumgeschnittenen Pfropfens durch Saugkräfte setzt wiederum den erörterten, gegenüber dem Stand der Technik wesentlich geringeren Pfropfendurchmesser voraus. Das Ablösen des Pfropfens durch Saugkräfte kann dadurch geschehen, daß am proximalen Ende der Kanüle, gegebenenfalls im Adapter eine vor Nutzung unverletzte, nach Durchstechen durch das Stilett selbstschließende Dichtungsscheibe angeordnet ist oder aber dadurch, daß ein gegebenenfalls zur Aufnahme eines Handdrehgriffs vorgesehener Adapter am proximalen Ende der Kanüle ebenfalls als Adapter zur Aufnahme einer Saugspritze ausgebildet ist, insbesondere nach WO-OS 86/06951 deren Inhalt zum Gegenstand der vorliegenden Offenbarung gemacht wird.

In weiterer Ausbildung ist in ansich bekannter Weise vorgesehen, daß das distale Ende der Kanüle abgeschrägt und mit einer Schneidkante versehen ist, wobei das abgeschrägte distale Stirnende der Kanüle derart mit einer Schneide auf dem Innenumfang der Wandung ausgebildet ist, daß die Wandung mit einer von außen schräg distal nach vorne und nach innen zulaufenden geschliffenen Schneidflanke versehen ist. Kanüle und Stilett bestehen im übrigen aus stabilem Metall, vorzugsweise Edelstahl. Vorzugsweise ist weiter vorgesehen, daß das distale stirnseitige Ende der Kanüle unter einem Winkel ungleich 90 Grad zur Kanülenachse gerichtet ist. Hierdurch wird das Angreifen des Gewindes am Knochen und das Eindrehen weiter unterstützt.

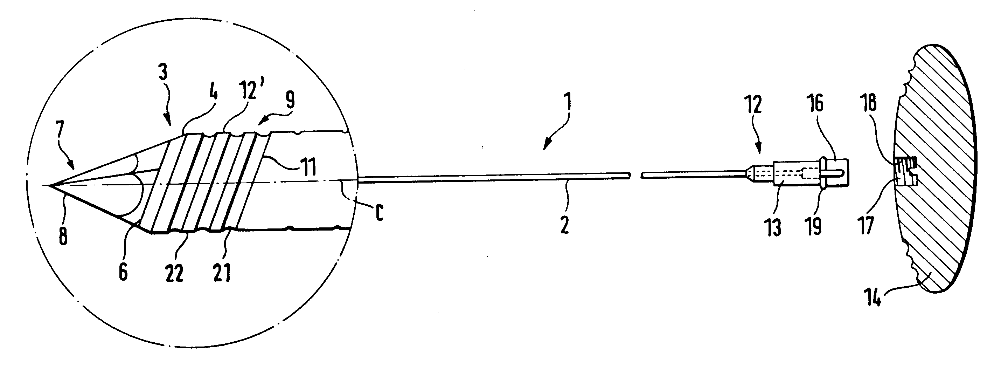

Weitere Vorteile und Merkmale der Erfindung ergeben sich aus den Ansprüchen und aus der nachfolgenden Beschreibung, in der ein Ausführungsbeispiel der erfindungsgemäßen Biopsienadel unter Bezugnahme auf die Zeichnung im einzelnen erläutert ist.

Dabei zeigt die einzige Figur:
eine Seitenansicht der erfindungsgemäßen Biopsienadel mit vergrößertem distalen Bereich A sowie zur Verwendung mit ihr vorgesehenem Handdrehgriff.

Die dargestellte Biopsienadel 1 weist eine äußere Kanüle 2 aus Stahl auf, die ein abgeschrägtes distales Ende 3 hat. Das distale Ende 3 ist durch eine konisch zulaufende äußere Schneidflanke 4 mit einer scharfen Schneidkante 6 auf dem Innenumfang des Mantels der Kanüle 2 versehen.

In der Kanüle 2 ist ein Stilett 7 geführt, das eine distale Spitze 8 mit einem Trokarschliff - also mit mindestens drei Flanken - aufweist und über die Höhe der Spitze 8 aus dem distalen Ende 3 der Kanüle 2 herausragt.

Nahe seinem distalen Ende 3 ist die Kanüle 2 mit einem zylindrischen Außengewinde 9 versehen. Im dargestellten Ausführungsbeispiel besteht das Außengewinde 9 aus drei Einzelwindungen 11 mit einer Gesamtgewindehöhe von ca. 5mm, die zur Erzielung der angestrebten axialen Führung vollauf ausreichen. Die Gewindesteigung liegt in der Größenordnung der Nutbreite bis vorteilhafterweise beim doppelten derselben, wie im dargestellten Beispiel. Die Tiefe der Gewindenut liegt vorzugsweise in der Größenordnung der Breite der Nut oder darunter, vorzugsweise bis zur Hälfte der Breite. Wichtig ist, daß das Außengewinde überschliffen ist, d.h. entgratet ist, so daß über den Außenumfang 12 der Kanüle 2 im Bereich der Windungen 11 keine Grate vorstehen. Das Gewinde ist durch den Überschliff derart ausgebildet, daß zwischen zwei benachbarten Windungsnuten 21 ein Gewindezahnbereich 22 verbleibt, der eine endliche Erstreckung in axialer Richtung, also in Richtung der Längsachse C aufweist, also insbesondere koaxial gerichtet ist.

Kanüle 2 und Stilett 7 bestehen aus Metall, vorzugsweise Edelstahl. Im dargestellten Ausführungsbeispiel weist die Kanüle 2 an ihrem proximalen Ende 12 einen Adapter 13, wie einen Luer Adapter zum Aufsetzen eines Handdrehgriffs 14,eines Motors und/oder einer Saugspritze (nicht dargestellt; nach Entfernen des Handdrehgriffs 14) auf. Stattdessen könnte der Handdrehgriff auch integral mit dem proximalen Ende 12 bzw. dem dort vorgesehenen Adapter ausgebildet sein. Insbesondere könnte auch vorgesehen sein, daß die Nadel 7, die mit einem dritten Teil 16 versehen ist, durch den Drehgriff 14 selbst in die Kanüle 2 einführbar ist.

Der Handdrehgriff 14 weist ein Innengewinde 18 in einer Ausnehmung 17 auf, der den Radialflansch 19 des Luer-Adapters hintergreift und derart eine sichere Verbindung zwischen Handgriff 14 und Adapter 13 und damit der Kanüle 2 herstellt.

In gleicher Weise kann an dem Adapter 13 ein Antriebsmotor zum motorischen Drehen der Kanüle 2 angebracht werden, dessen Einsatz die oben genannten Vorteile bedingt, insbesondere ein leichtes Eindringen in alle Knochen ermöglicht.

Die Länge der freien Kanüle, ohne mit ihr verbundenem Adapter 3 ist relativ kurz gewählt, um Gefahren eines Biegens oder Abknickens der Nadel weitgehend zu reduzieren und liegt unter 20 mm, insbsondere bei etwa 100 mm. Die Gesamtlänge der Kanüle liegt bei 120 bis 140 mm, wobei zwischen 15 und 20 mm vom Adapter 13 umschlossen sind.

Die Stärke der Kanüle 2 kann durch die durch das Gewinde 9 gegebene Führung relativ gering gewählt werden. Der Außendurchmesser der Kanüle 2 beträgt daher weniger als 2 mm, vorzugsweise zwischen 1 und 1,5 mm. Der lichte oder Innendurchmesser der Kanüle liegt unter 1,5, vorzugsweise zwischen 0,7 und 0,2 mm. Die Wandstärken der Kanüle liegen unter 0,3, vorzugsweise im Bereich von 0,1 bis 0,2 mm.

Die erfindungsgemäße Biopsienadel wird mit eingeschobenem Stilett in an sich bekannter Weise, gegebenenfalls zunächst einfach perkutan eingestochen - ohne daß eine vorherige Hautinzision gemacht wird -anschließend bis zum Knochen vor und eventuell in diesem, gegebenenfalls unter Zuhilfenahme eines bekannten Biopsie-Teakholz-Hammers, bis in den zu untersuchenden Bereich axial eingetrieben. Dann wird das Stilett 7 aus der Kanüle 2 herausgezogen und der Drehhandgriff 14 oder - noch vorteilhafter - ein Antriebsmotor auf den Adapter 13 der Kanüle 2 aufgesetzt. Sodann erst wird die Kanüle 2 mittels des Drehgriffs 14 oder eines Motors weiter im Knochen eingedreht, wobei die Vorschubbewegung durch das Gewinde 9 unterstützt wird. Ein vorheriges Herausschneiden von subkutanem Gewebe erfolgt, im Gegensatz zum Stande der Technik nicht. Da nun das distale Ende 3 offen ist, kann in dieses ein aus dem umgebenden Knochenmaterial durch die Schneide 6 herausgeschnittener Materialpropfen in die Hohlnadel 2 eindringen. Bevor die Hohlnadel wieder herausgezogen wird, gegebenenfalls nach Entfernen des Handgriffs 14 oder Motors, wird auf dem Adapter 13 eine Saugspritze aufgesetzt und aufgezogen, gegebenenfalls unter Zuhilfenahme einer den Kolben der Spritze herausdrückenden Feder, so daß innerhalb der Hohlnadel 2 ein Unterdruck entsteht, der den in ihr befindlichen Materialpropfen insbesondere vom umgebenden Material abreißen läßt, beim Herausziehen der Hohlnadel 2 aus dem Körperinneren festhält, so daß er sicher innerhalb der Hohlnadel 2 nach außen gebracht werden kann. Die Hohlnadel wird lediglich axial, ohne Drehungen herausgezogen. Dies ist durch das überschliffene Gewinde möglich und reduziert wesentlich die Gefahr von Traumata. Anschließend kann der Pfropfen, nach Entfernen der Spritze, mit einem Stift, beispielsweise dem Stilett 7 zum distalen Ende 3 der Hohlnadel 2 herausgestoßen und der weiteren Untersuchung zugeführt werden. Statt einer zusätzlichen Spritze könnte der Unterdruck in ansich bekannter Weise auch dadurch erzeugt werden, daß im Adapter 13 ein vor Benutzung der Biopsienadel 1 unzerstochenes Dichtungsplättchen vorgesehen ist, das zur Durchführung der Probenentnahme durch das Stilett 7 durchstochen wird, dieses dicht umschließt, so daß bei Entfernen des Stiletts in der Kanüle 2 ein Unterdruck entsteht, der nach vollständigem Entfernen des Stiletts aufgrund der Eigenelastizität der Dichtungsscheibe, die sich nach Entfernen des Stiletts wieder vollständig schließt, weitgehend aufrecht erhalten wird und ebenfalls den Material pfropfen im distalen Ende 3 der Hohlnadel festhalten kann, so daß er herausgezogen werden kann (WO-OS 86/06951).

**Patentansprüche**

1. Biopsienadel (1) zur Knochenbiopsie, insbesondere durch Einbringen der Nadel in und gegebenenfalls durch die Knochengrundsubstanz, mit einer distal stirnseitig geschliffenen Kanüle (2) und einem in dieser geführten Stilett (7), wobei die Kanüle (2) an ihrem distalen Ende (3) ein Außengewinde (9) aufweist, dadurch gekennzeichnet, daß das Außengewinde derart überschliffen ist, daß die Wandungen zweier benachbarter Gewindeteile oder -nuten nicht über eine Gewinde-Schneidkante ineinander übergehen, sondern über einen Außenmantelbereich mit endlicher Erstreckung in axialer Richtung, dessen Außendurchmesser nicht größer als der Außendurchmesser der sonstigen, im wesentlichen zylindrischen Kanülen ist.

2. Biopsienadel nach Anspruch 1, dadurch gekennzeichnet, daß das Stilett (7) eine distale Spitze (8) mit Trokarschliff aufweist.

3. Biopsienadel nach Anspruch 1 oder 2, dadurch gekennzeichnet, daß das Gewinde (9) eine Doppelwendel aufweist.

4. Biopsienadel nach einem der Ansprüche 1 bis 3, dadurch gekennzeichnet, daß das Gewinde (9) auf einem zylindrischen Mantel ausgebildet ist.

5. Biopsienadel nach einem der vorangehenden Ansprüche, dadurch gekennzeichnet, daß die Kanüle fest mit einem Handdrehgriff (14) versehen ist.

6. Biopsienadel nach einem der Ansprüche 1 bis 5, dadurch gekennzeichnet, daß die Kanüle (2) mit einem Adapter (13) für ein Antriebsteil (14) versehen ist.

7. Biopsienadel nach einem der Ansprüche 1 bis 6, dadurch gekennzeichnet, daß ein motorischer Antrieb anbringbar ist.

8. Biopsienadel nach einem der Ansprüche 5 bis 7, dadurch gekennzeichnet, daß am proximalen Ende (10) der Kanüle (2), gegebenenfalls im Adapter (13) eine vor Nutzung unverletzter, nach Durchstechen durch das Stilett (7) selbstschließende Dichtungsscheibe angeordnet ist.

9. Biopsienadel nach Anspruch 6, dadurch gekennzeichnet, daß der Adapter (13) zur Verbindung mit einer Saugspritze ausgebildet ist.

10. Biopsienadel nach einem der vorangehenden Ansprüche, dadurch gekennzeichnet, daß der Außendurchmesser der Kanüle kleiner als 2 mm ist.

11. Biopsienadel nach einem der Ansprüche 1 bis 10, dadurch gekennzeichnet, daß der Kanüleninnendurchmesser zwischen 0,7 und 1,2 mm liegt.

12. Biopsienadel nach Anspruch 10 oder 11, dadurch gekennzeichnet, daß die Wandstärke der Kanüle im Bereich von 0,1 bis 0,2 mm liegt.

13. Biopsienadel nach einem der vorangehenden Ansprüche, dadurch gekennzeichnet, daß das distale Ende (3) der Kanüle (2) abgeschrägt und mit einer Schneidkante (6) versehen ist.

14. Biopsienadel nach einem der vorangehenden Ansprüche, dadurch gekennzeichnet, daß das distale stirnseitige Ende der Kanüle (2) unter einem Winkel ungleich 90 Grad zur Kanülachse gerichtet ist.

15. Biopsienadel nach einem der vorangehenden Ansprüche, gekennzeichnet durch eine am proximalen Ende der Kanüle (2) ansetzbare Saugspritze.

**Claims**

1. Biopsy needle (1) for bone biopsy, particularly by introducing the needle into and optionally through the basic bone substance, with a distal end ground cannula (2) and a stylet (7) guided therein, the cannula (2) having at its distal end (3) an outer thread (9), characterized in that the outer thread is over-round in such a way that the walls of two adjacent thread parts or grooves do not pass into one another via a thread cutting edge and instead pass via an outer jacket area with finite extension in the axial direction, whose external diameter is no larger than the external diameter of the remaining, substantially cylindrical cannula.

2. Biopsy needle according to claim 1, characterized in that the stylet (7) has a distal tip (8) with a trocar grinding pattern.

3. Biopsy needle according to claims 1 or 2, characterized in that the thread (9) has a wound coil.

4. Biopsy needle according to any one of the claims 1 to 3, characterized in that the thread (9) is formed on a cylindrical jacket.

5. Biopsy needle according to any one of the preceding claims, characterized in that the cannula is firmly provided with a control grip.

6. Biopsy needle according to any one of the claims 1 to 5, characterized in that the cannula (2) is provided with an adaptor (13) for a drive part (14).

7. Biopsy needle according to any one of the claims 1 to 6, characterized in that a motor drive can be fitted.

8. Biopsy needle according to any one of the claims 5 to 7, characterized in that on the proximal end (10) of the cannula (2) and optionally in the adaptor (13) is provided a sealing disk, which cannot be damaged by use and which self-seals following perforation by the stylet (7).

9. Biopsy needle according to claim 6, characterized in that the adaptor (13) is constructed for connection to a suction syringe.

10. Biopsy needle according to any one of the preceding claims, characterized in that the external diameter of the cannula is smaller than 2 mm.

11. Biopsy needle according to any one of the claims 1 to 10, characterized in that the cannula internal diameter is between 0.7 and 1.2 mm.

12. Biopsy needle according to claims 10 or 11, characterized in that the wall thickness of the cannula is in the range 0.1 to 0.2 mm.

13. Biopsy needle according to any one of the preceding claims, characterized in that the distal end (3) of the cannula (2) is bevelled and is provided with a cutting edge (6).

14. Biopsy needle according to any one of the preceding claims, characterized in that the distal end of the cannula (2) is directed at an angle not equal to 90° to the cannula axis.

15. Biopsy needle according to any one of the preceding claims, characterized by a suction syringe which can be fitted to the proximal end of the cannula (2).

**Revendications**

1. Aiguille de biopsie (1) pour la biopsie osseuse, en particulier pour l'introduction de l'aiguille dans et éventuellement au travers de la substance osseuse de base, comprenant une canule (2) taillée du côté frontal distal et un stylet (7) guidé dans celle-ci, la canule (2) étant munie à son extrémité distale (3) d'un filetage extérieur (9), **caractérisée en ce** que le filetage extérieur est surmeulé de telle façon que les flancs de deux éléments ou rainures de filet voisins se raccordent non pas par l'intermédiaire d'une arête coupante mais par l'intermédiaire d'une section d'enveloppe extérieure d'une extension finie dans la direction axiale dont le diamètre extérieur ne dépasse pas le diamètre extérieur du reste de la canule sensiblement cylindrique.

2. Aiguille de biopsie selon la revendication 1, caractérisée en ce que le stylet (7) présente une pointe distale (8) taillée en trocart.

3. Aiguille de biopsie selon l'une des revendications 1 ou 2, caractérisée en ce que le filetage (9) présente une double spirale.

4. Aiguille de biopsie selon l'une des revendications 1 à 3, caractérisée en ce que le filetage (9) est conformé sur une enveloppe cylindrique.

5. Aiguille de biopsie selon l'une des revendications précédentes, caractérisée en ce que la canule est solidarisée avec une poignée tournante (14).

6. Aiguille de biopsie selon l'une des revendications 1 à 5, caractérisée en ce que la canule (2) est équipée d'un adaptateur (13) pour un élément d'entraînement (14).

7. Aiguille de biopsie selon l'une des revendications 1 à 6, caractérisée en ce qu'elle peut être équipée d'un entraînement motorisé.

8. Aiguille de biopsie selon l'une des revendications 5 à 7, caractérisée en ce qu'à l'extrémité proximale (10) de la canule (2) est disposé, éventuellement dans l'adaptateur (13), un disque d'étanchéité intact avant l'utilisation et se refermant automatiquement après le percement par le stylet (7).

9. Aiguille de biopsie selon la revendication 6, caractérisée en ce que l'adaptateur (13) est conformé pour la mise en place d'une seringue aspirante.

10. Aiguille de biopsie selon l'une des revendications précédentes, caractérisée en ce que le diamètre extérieur de la canule est inférieur à 2 mm.

11. Aiguille de biopsie selon l'une des revendications 1 à 10, caractérisée en ce que le diamètre intérieur de la canule se situe entre 0,7 et 1,2 mm.

12. Aiguille de biopsie selon l'une des revendications 10 ou 11, caractérisée en ce que l'épaisseur de paroi de la canule est de l'ordre de 0,1 à 0,2 mm.

13. Aiguille de biopsie selon l'une des revendications précédentes, caractérisée en ce que l'extrémité distale (3) de la canule (2) est biseautée et munie d'une arête coupante (6).

14. Aiguille de biopsie selon l'une des revendications précédentes, caractérisée en ce que l'extrémité frontale distale de la canule (2) forme avec l'axe de la canule un angle différent de 90 degrés.

15. Aiguille de biopsie selon l'une des revendications précédentes, caractérisée en ce qu'elle comprend une seringue aspirante qui peut être montée sur l'extrémité proximale de la canule (2).